# EUROPEAN PATENT APPLICATION

(11) **EP 3 699 267 A1**
(43) Date of publication of application: **26.08.2020**
(21) Application number: 18868426.0
(22) Date of filing: 17.10.2018
(51) Int. Cl.: C12N 5/10, A61K 35/545, A61L 27/36, A61L 27/38, A61P 21/00, C12Q 1/02, G01N 33/15, G01N 33/50, C07K 14/47, C12N 15/09

(54) **METHOD FOR OBTAINING ARTIFICIAL NEUROMUSCULAR JUNCTION FROM PLURIPOTENT STEM CELLS**

(30) Priority: 17.10.2017 JP 2017201364
(71) Applicant: Kyoto University, Kyoto-shi, Kyoto 606-8501 (JP)
(72) Inventor: SAITO, Megumu, Kyoto-shi Kyoto 606-8501 (JP); YOSHIDA, Michiko, Kyoto-shi Kyoto 606-8501 (JP); LIN, Chuang-Yu, Kyoto-shi Kyoto 606-8501 (JP)
(74) Representative: Hoffmann Eitle
(86) International application number: PCT/JP2018/038690
(87) International publication number: WO 2019/078263

(57) **Abstract**

The present invention addresses the problem of providing a method for easily and efficiently preparing a functional and matured neuromuscular junction (NMJ) in vitro. This problem is solved by a method for producing an artificial neuromuscular junction, said method comprising: (i) a step for transiently expressing MyoD in pluripotent stem cells to induce myogenic differentiation; and (ii) a step for culturing the cells obtained in (i) in a medium containing a neurotrophic factor to induce neuronal differentiation.

SEQUENCE LISTING

<110>  Kyoto University

<120>  A method for obtaining artificial neuromuscular junction from
pluripotent stem cell

<130>  5918-18496

<150>  JP 2017-201364
<151>  2017-10-17

<160>  2

<170>  PatentIn version 3.5


<210>  1
<211>  1823
<212>  DNA
<213>  Homo sapiens


<220>
<221>  CDS
<222>  (221)..(1183)

<400>  1
gagaagctag gggtgaggaa gccctggggc gctgccgccg ctttccttaa ccacaaatca      60
ggccggacag gagagggagg ggtgggggac agtgggtggg cattcagact gccagcactt     120
tgctatctac agccggggct cccgagcggc agaaagttcc ggccactctc tgccgcttgg     180
gttgggcgaa gccaggaccg tgccgcgcca ccgccaggat atg gag cta ctg tcg       235
                                            Met Glu Leu Leu Ser
                                            1               5
cca ccg ctc cgc gac gta gac ctg acg gcc ccc gac ggc tct ctc tgc       283
Pro Pro Leu Arg Asp Val Asp Leu Thr Ala Pro Asp Gly Ser Leu Cys
                10                  15                  20
tcc ttt gcc aca acg gac gac ttc tat gac gac ccg tgt ttc gac tcc       331
Ser Phe Ala Thr Thr Asp Asp Phe Tyr Asp Asp Pro Cys Phe Asp Ser
            25                  30                  35
ccg gac ctg cgc ttc ttc gaa gac ctg gac ccg cgc ctg atg cac gtg       379
Pro Asp Leu Arg Phe Phe Glu Asp Leu Asp Pro Arg Leu Met His Val
        40                  45                  50
ggc gcg ctc ctg aaa ccc gaa gag cac tcg cac ttc ccc gcg gcg gtg       427
Gly Ala Leu Leu Lys Pro Glu Glu His Ser His Phe Pro Ala Ala Val
    55                  60                  65
cac ccg gcc ccg ggc gca cgt gag gac gag cat gtg cgc gcg ccc agc       475
His Pro Ala Pro Gly Ala Arg Glu Asp Glu His Val Arg Ala Pro Ser
70                  75                  80                  85
ggg cac cac cag gcg ggc cgc tgc cta ctg tgg gcc tgc aag gcg tgc       523
Gly His His Gln Ala Gly Arg Cys Leu Leu Trp Ala Cys Lys Ala Cys
                90                  95                  100
aag cgc aag acc acc aac gcc gac cgc cgc aag gcc gcc acc atg cgc       571
Lys Arg Lys Thr Thr Asn Ala Asp Arg Arg Lys Ala Ala Thr Met Arg
            105                 110                 115
gag cgg cgc cgc ctg agc aaa gta aat gag gcc ttt gag aca ctc aag       619
Glu Arg Arg Arg Leu Ser Lys Val Asn Glu Ala Phe Glu Thr Leu Lys
        120                 125                 130
cgc tgc acg tcg agc aat cca aac cag cgg ttg ccc aag gtg gag atc       667
Arg Cys Thr Ser Ser Asn Pro Asn Gln Arg Leu Pro Lys Val Glu Ile
    135                 140                 145
ctg cgc aac gcc atc cgc tat atc gag ggc ctg cag gct ctg ctg cgc       715
Leu Arg Asn Ala Ile Arg Tyr Ile Glu Gly Leu Gln Ala Leu Leu Arg

150                 155                 160                 165
gac cag gac gcc gcg ccc cct ggc gcc gca gcc gcc ttc tat gcg ccg      763
Asp Gln Asp Ala Ala Pro Pro Gly Ala Ala Ala Ala Phe Tyr Ala Pro
                170                 175                 180
ggc ccg ctg ccc ccg ggc cgc ggc ggc gag cac tac agc ggc gac tcc      811
Gly Pro Leu Pro Pro Gly Arg Gly Gly Glu His Tyr Ser Gly Asp Ser
            185                 190                 195
gac gcg tcc agc ccg cgc tcc aac tgc tcc gac ggc atg atg gac tac      859
Asp Ala Ser Ser Pro Arg Ser Asn Cys Ser Asp Gly Met Met Asp Tyr
        200                 205                 210
agc ggc ccc ccg agc ggc gcc cgg cgg cgg aac tgc tac gaa ggc gcc      907
Ser Gly Pro Pro Ser Gly Ala Arg Arg Arg Asn Cys Tyr Glu Gly Ala
    215                 220                 225
tac tac aac gag gcg ccc agc gaa ccc agg ccc ggg aag agt gcg gcg      955
Tyr Tyr Asn Glu Ala Pro Ser Glu Pro Arg Pro Gly Lys Ser Ala Ala
230                 235                 240                 245
gtg tcg agc cta gac tgc ctg tcc agc atc gtg gag cgc atc tcc acc     1003
Val Ser Ser Leu Asp Cys Leu Ser Ser Ile Val Glu Arg Ile Ser Thr
                250                 255                 260
gag agc cct gcg gcg ccc gcc ctc ctg ctg gcg gac gtg cct tct gag     1051
Glu Ser Pro Ala Ala Pro Ala Leu Leu Leu Ala Asp Val Pro Ser Glu
            265                 270                 275
tcg cct ccg cgc agg caa gag gct gcc gcc ccc agc gag gga gag agc     1099
Ser Pro Pro Arg Arg Gln Glu Ala Ala Ala Pro Ser Glu Gly Glu Ser
        280                 285                 290
agc ggc gac ccc acc cag tca ccg gac gcc gcc ccg cag tgc cct gcg     1147
Ser Gly Asp Pro Thr Gln Ser Pro Asp Ala Ala Pro Gln Cys Pro Ala
    295                 300                 305
ggt gcg aac ccc aac ccg ata tac cag gtg ctc tga ggggatggtg          1193
Gly Ala Asn Pro Asn Pro Ile Tyr Gln Val Leu
310                 315                 320
gccgcccacc cgcccgaggg atggtgcccc tagggtccct cgcgcccaaa agattgaact   1253
taaatgcccc cctcccaaca gcgctttaaa agcgacctct cttgaggtag gagaggcggg   1313
agaactgaag tttccgcccc cgccccacag ggcaaggaca cagcgcggtt ttttccacgc   1373
agcacccttc tcggagaccc attgcgatgg ccgctccgtg ttcctcggtg ggccagagct   1433
gaaccttgag gggctaggtt cagctttctc gcgccctccc ccatgggggt gagaccctcg   1493
cagacctaag ccctgccccg ggatgcaccg gttatttggg ggggcgtgag acccagtgca   1553
ctccggtccc aaatgtagca ggtgtaaccg taacccaccc ccaacccgtt tcccggttca   1613
ggaccacttt ttgtaatact tttgtaatct attcctgtaa ataagagttg ctttgccaga   1673
gcaggagccc ctggggctgt atttatctct gaggcatggt gtgtggtgct acagggaatt   1733
tgtacgttta taccgcaggc gggcgagccg cgggcgctcg ctcaggtgat caaaataaag   1793
gcgctaattt ataaaaaaaa aaaaaaaaaa                                    1823

<210>  2
<211>  320
<212>  PRT
<213>  Homo sapiens

<400>  2
Met Glu Leu Leu Ser Pro Pro Leu Arg Asp Val Asp Leu Thr Ala Pro
1               5                   10                  15
Asp Gly Ser Leu Cys Ser Phe Ala Thr Thr Asp Asp Phe Tyr Asp Asp
            20                  25                  30
Pro Cys Phe Asp Ser Pro Asp Leu Arg Phe Phe Glu Asp Leu Asp Pro
        35                  40                  45
Arg Leu Met His Val Gly Ala Leu Leu Lys Pro Glu Glu His Ser His
    50                  55                  60
Phe Pro Ala Ala Val His Pro Ala Pro Gly Ala Arg Glu Asp Glu His
65                  70                  75                  80
Val Arg Ala Pro Ser Gly His His Gln Ala Gly Arg Cys Leu Leu Trp
                85                  90                  95
Ala Cys Lys Ala Cys Lys Arg Lys Thr Thr Asn Ala Asp Arg Arg Lys

                100                 105                 110
Ala Ala Thr Met Arg Glu Arg Arg Arg Leu Ser Lys Val Asn Glu Ala
        115                 120                 125
Phe Glu Thr Leu Lys Arg Cys Thr Ser Ser Asn Pro Asn Gln Arg Leu
    130                 135                 140
Pro Lys Val Glu Ile Leu Arg Asn Ala Ile Arg Tyr Ile Glu Gly Leu
145                 150                 155                 160
Gln Ala Leu Leu Arg Asp Gln Asp Ala Ala Pro Pro Gly Ala Ala Ala
                165                 170                 175
Ala Phe Tyr Ala Pro Gly Pro Leu Pro Pro Gly Arg Gly Gly Glu His
            180                 185                 190
Tyr Ser Gly Asp Ser Asp Ala Ser Ser Pro Arg Ser Asn Cys Ser Asp
        195                 200                 205
Gly Met Met Asp Tyr Ser Gly Pro Pro Ser Gly Ala Arg Arg Arg Asn
    210                 215                 220
Cys Tyr Glu Gly Ala Tyr Tyr Asn Glu Ala Pro Ser Glu Pro Arg Pro
225                 230                 235                 240
Gly Lys Ser Ala Ala Val Ser Ser Leu Asp Cys Leu Ser Ser Ile Val
                245                 250                 255
Glu Arg Ile Ser Thr Glu Ser Pro Ala Ala Pro Ala Leu Leu Leu Ala
            260                 265                 270
Asp Val Pro Ser Glu Ser Pro Pro Arg Arg Gln Glu Ala Ala Ala Pro
        275                 280                 285
Ser Glu Gly Glu Ser Ser Gly Asp Pro Thr Gln Ser Pro Asp Ala Ala
    290                 295                 300
Pro Gln Cys Pro Ala Gly Ala Asn Pro Asn Pro Ile Tyr Gln Val Leu
305                 310                 315                 320

## Description

### Technical Field

The present invention relates to a method for preparing a neuromuscular junction *in vitro* utilizing a pluripotent stem cell, an artificial neuromuscular junction obtained by the method, and use thereof.

### Background Art

The neuromuscular junction (NMJ) is a junction between a motor neuron terminal and a muscle, where acetylcholine released from the neuron terminal causes muscle contraction.

NMJ formation and maintenance is a multistep process that requires a controlled and continuous interaction between NMJ components such as nerve cells and muscle cells. It is known that pathogenic processes of NMJ-related diseases involve not only NMJ dysfunction but also dysfunction at the stage of NMJ development. An NMJ derived from a human induced pluripotent stem cell (hiPSC), which reproduces formation and maintenance of an NMJ *in vivo,* can be an excellent model for elucidating the pathophysiology of such diseases and discovering therapeutics therefor. In recent years, several attempts have been made to reconstitute an NMJ *in vitro.* For example, although Non Patent Literature 1 and Non Patent Literature 2 each show that an NMJ model obtained by co-culture of a hiPSC-derived motor neuron (MN) and a primary muscle cell or co-culture of a hiPSC-derived MN and a hiPSC-derived muscle cell forms an acetylcholine receptor (AChR) assembly, such a model has not reached maturity in terms of morphology and function, and was insufficient as an NMJ model.

### Citation List

### Non Patent Literature

Non Patent Literature 1: Steinbeck JA. et, al. Cell Stem Cell. 2016 Jan.7; 18(1): 134-43
Non Patent Literature 2: Demestra M, et, al. Stem Cell Res. 2015 Sep; 15(2): 328-36

### Summary of Invention

An object of the present invention is to provide a method for preparing a functional and mature NMJ *in vitro* easily and efficiently.

In order to solve the above problem, the present inventors have conducted intensive studies and found that, by temporarily expressing MyoD (myogenic differentiation) gene in pluripotent stem cells and then switching the culture condition from muscle induction to nerve induction, the pluripotent stem cells can be efficiently induced to differentiate into functional and mature NMJ comprising motor neurons, muscle cells (myotubes), and Schwann cells, thereby completed the present invention.

The present invention provides the following.
[1] A method for producing an artificial neuromuscular junction, comprising the steps of:
   (i) transiently expressing MyoD in pluripotent stem cells to induce myogenic differentiation, and
   (ii) culturing the cells obtained in the step (i) in a medium comprising a neurotrophic factor(s) to induce neural differentiation.
[2] The method according to [1], wherein the transient expression of MyoD in the step (i) is a drug-induced expression.
[3] The method according to [1] or [2], wherein the neurotrophic factors in the step (ii) are Glial cell line-derived neurotrophic factor (GDNF), Brain-derived neurotrophic factor (BDNF), and Neurotrophin 3 (NT-3).
[4] The method according to any one of [1] to [3], wherein the step (i) is carried out for from 4 to 20 days.
[5] The method according to any one of [1] to [4], wherein the step (ii) is carried out for from 20 to 120 days.
[6] The method according to any one of [1] to [5], wherein the artificial neuromuscular junction comprises motor neuron, muscle cells, and Schwann cells.
[7] The method according to any one of [1] to [6], wherein the pluripotent stem cells are induced pluripotent stem cells.
[8] The method according to [7], wherein the pluripotent stem cells are human induced pluripotent stem cells.
[9] A cell population comprising a motor neuron, a muscle cell, and a Schwann cell, each induced from a pluripotent stem cell.
[10] A pharmaceutical composition comprising the cell population according to [9].
[11] A method for screening or evaluating a therapeutic agent for a disease caused by a disorder of a neuromuscular junction, comprising the steps of:
   adding a test substance to the cell population according to [9] and culturing the cell population, and
   after culturing, evaluating muscle contraction or calcium concentration in the cells.
[12] A method for screening or evaluating a therapeutic agent for a disease caused by a disorder of a neuromuscular junction, comprising a process for producing an artificial neuromuscular junction, said process comprising the steps of (i) transiently expressing MyoD in pluripotent stem cells to induce myogenic differentiation, and (ii) inducing neural differentiation by culturing the cells obtained in the step (i) in a medium comprising a neurotrophic factor(s),
   wherein a test substance exists in the step(s) (i) and/or (ii), and
   wherein the test substance is evaluated by analyzing morphology or cell composition of the obtained artificial neuromuscular junction.

According to the present invention, NMJ can be obtained efficiently in an *in vitro* system. The formed NMJ has the characteristics of NMJ including AChR and exhibits NMJ-mediated muscle contraction by nerve.

Schwann cells have been reported to be involved in maturation of nerve terminals, maturation of AChR clusters, and maintenance of NMJ (The Journal of Neuroscience, September 21, 2016, 36 (38): 9770-9781), and according to the method of the present invention, more mature and functional NMJ comprising Schwann cells is obtained.

According to the method of the present invention, human NMJ can be formed in a short period of about 30 days, and can be cultured and maintained for a long period of about 100 days after the formation.

According to the method of the present invention, an *in vivo* synapse formation process is reproduced which enables to understand NMJ synapse formation, and therefore, the method is useful for elucidation of the onset of NMJ-related diseases, screening of candidate therapeutic agents, and the like.

### Brief Description of the Drawings

Fig. 1A: *In vitro* hNMJ formation and morphological analysis. Scheme of *in vitro* hNMJ formation from hiPSCs.
Fig. 1B: *In vitro* hNMJ formation and morphological analysis (photograph). Immunostaining of *in vitro* hNMJ. Morphological change of AChR clustering from plaque-like to pretzel-like. (Right) Detection of ε subunit of AChR. Scale bar without labeling = 10 µm.
Fig. 1C: *In vitro* hNMJ formation and morphological analysis (photograph). Immunostaining of *in vitro* hNMJ. Detection of MN differentiation markers. Scale bar without labeling = 10 µm.
Fig. 1D: *In vitro* hNMJ formation and morphological analysis (photograph). Immunostaining of *in vitro* hNMJ. Detection of myogenic cell differentiation markers. Scale bar without labeling = 10 µm.
Fig. 1E: *In vitro* hNMJ formation and morphological analysis (photograph). Immunostaining of *in vitro* hNMJ. Change in neurofilament density from early to late stage.
Fig. 1F: *In vitro* hNMJ formation and morphological analysis (photograph). Immunostaining of *in vitro* hNMJ. Detection of Schwann cell marker. Scale bar without labeling = 10 µm.
Fig. 1G: *In vitro* hNMJ formation and morphological analysis (photograph). Electron microscopic analysis of *in vitro* hNMJ. SEM showing swelling of axon terminal connecting to myotube.
Fig. 1H: *In vitro* hNMJ formation and morphological analysis (photograph). Electron microscopic analysis of *in vitro* hNMJ. TEM showing synaptic vesicle at presynaptic site (arrowhead), active zone (arrow), and mitochondria accumulated at postsynaptic site.
Fig. 1I: *In vitro* hNMJ formation and morphological analysis (photograph). Electron microscopic analysis of *in vitro* hNMJ. Magnified axon terminal observed inside myotubes. Junctional fold shown as high electron density line.
Fig. 1J: *In vitro* hNMJ formation and morphological analysis (photograph). Electron microscopic analysis of *in vitro* hNMJ. Schwann cells covering terminal bouton.

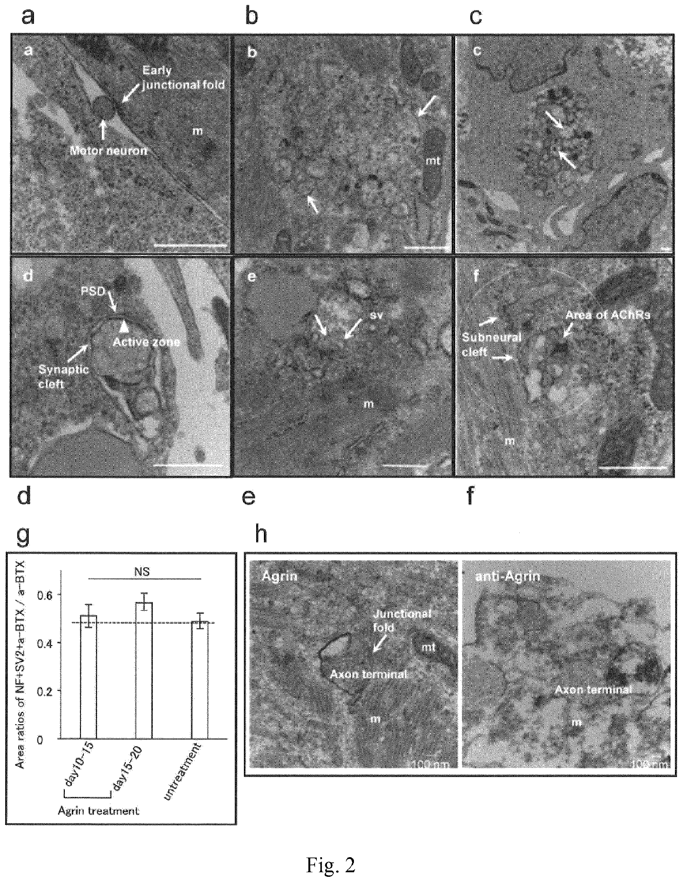

Fig. 2: Analysis of hNMJ developmental stage by TEM (photograph). (a) MN that distributes nerve to myotube (m). High electron density region observed on muscle plasma membrane. (b) Hyperbranched axon terminal observed in myotubes. High electron density region (arrow) is located at each axon terminal. Mitochondria (mt) was found nearby. (c) Necrosis (arrow) observed at axon terminal. (d) Active zone (arrowhead), juxtaposed postsynaptic density (PSD, arrow), and synaptic cleft observed therebetween. (e) Synaptic vesicle (sv) docks in active zone (arrow). (f) Subneural cleft that is surrounded by sarcolemma and in which AChR resides. (g) Ratio of NMJ region increased by agrin treatment. (h) Agrin treatment: Myotube was well confined at fold of junction that bounds axon terminal. Mitochondria is found near junction region. Anti-aglin treatment: Only myotube remnant and degraded axon terminal were left in culture. One-way ANOVA was carried out for statistical analysis.
Fig. 3A: Functional analysis of *in vitro* hNMJ on day 20 of NMJ differentiation (upper left is photograph). Detection of transient Ca²⁺ elevation and concomitant myotube movement detection in two regions of interest (ROI). Myotubes contracted in response to elevated Ca²⁺.
Fig. 3B: Functional analysis of *in vitro* hNMJ on day 20 of NMJ differentiation (upper left is photograph). Ca imaging at five ROIs. Transient Ca²⁺ elevation was enhanced by addition of calcium, and was stopped by curare treatment.
Fig. 3C: Functional analysis of *in vitro* hNMJ on day 20 of NMJ differentiation (top is photograph). Motion analysis at four ROIs shows propagation of myotube contraction.
Fig. 3D:Functional analysis of *in vitro* hNMJ on day 20 of NMJ differentiation. Fifteen minutes after gap junction inhibition by GAP27, myotube in four ROIs began to contract independently.
Fig. 4: Motion analysis of *in vitro* hNMJ culture by photoactivation (a, c, b below are photographs). (a) Neuronal cell of hNMJ culture expressing channel ChR2-EYFP. (b and c) Motion analysis of hNMJ culture. (b) Muscle contraction was caused only by blue light (488 nm). (c) Muscle contraction was inhibited by curare.
Fig. 5A: Detection and quantification of MN in hNMJ culture (the upper is photograph). Image and graph of MN-specific marker-positive cells on day 60 of culture (n = 2, mean ± SEM).
Fig. 5B: Detection and quantification of MN in hNMJ culture. Results of the number of HB9-positive cells and the number of Islet1-positive cells relative to the total number of cells on day 30 and day 60 of culture by flow cytometry (n = 3, mean ± SEM).
Fig. 6A: Differentiation of SMN protein-knock down iPSCs (201B7^{MYOD}-SMN^{KD}) to hNMJ (upper ones are photographs). Result of Western blot analysis of SMN protein, which is quantification result by gel image and signal intensity (n = 3, mean ± SEM).
Fig. 6B: Differentiation of SMN protein-knock down iPSCs (201B7^{MYOD}-SMN^{KD}) to hNMJ (upper ones are photographs). Images and results of analysis of cell area on NF+SV2 immunostaining and AChR immunostaining of 201B7^{MYOD}-SMN^{KD} cells (SMN KD) and 201B7^{MYOD} cells (Control) on day 30 of culture. Scale bar: 10 µm.
Fig. 6C: Differentiation of SMN protein-knock down iPSCs (201B7^{MYOD}-SMN^{KD}) to hNMJ (photographs). Scanning electron microscope images showing morphology of myotube and axon terminal in 201B7^{MYOD}-SMN^{KD} cells (SMN KD) and 201B7^{MYOD} cells (Control). Scale bar: 500 nm. Mf in the figures indicates myotube, and Ax indicates axon.
Fig. 6D: Differentiation of SMN protein-knock down iPSCs (201B7^{MYOD}-SMN^{KD}) to hNMJ (photographs). Transmission electron microscopy images showing myotube and axon terminal in 201B7^{MYOD}-SMN^{KD} cells (SMN KD) and 201B7^{MYOD} cells (Control). L indicates lipid droplet, Mt indicates mitochondria, Mf indicates myotube, SV indicates synaptic vesicle, Ax indicates axon, and Nu indicates nucleus.
Fig. 6E: Differentiation of SMN protein-knock down iPSCs (201B7^{MYOD}-SMN^{KD}) to hNMJ (upper ones are photographs). Images and graphs showing time course of myotube contraction in 201B7^{MYOD}-SMN^{KD} cells (SMN KD) and 201B7^{MYOD} cells (Control) on day 30 of culture.
Fig. 6F: Differentiation of SMN protein-knock down iPSCs (201B7^{MYOD}-SMN^{KD}) (photographs) to hNMJ. Bright-field image showing collapse of myotube during contraction in 201B7^{MYOD}-SMN^{KD} cells.

### Description of Embodiments

### < Method for Producing Artificial Neuromuscular junction>

The method for producing an artificial neuromuscular junction of the present invention comprises:
(i) transiently expressing MyoD in pluripotent stem cells to induce myogenic differentiation, and
(ii) inducing neural differentiation by culturing the cells obtained in (i) in a medium comprising a neurotrophic factor(s).

### <Artificial Neuromuscular Junction>

An artificial neuromuscular junction refers to a neuromuscular junction induced from pluripotent stem cells *in vitro.* The neuromuscular junction refers to a structure in which acetylcholine is released from a protruding end of a motor neuron and can be received by a receptor present in a myotube cell. Presence of a neuromuscular junction can be confirmed by colocalization of a synaptic vesicle protein expressed by a motor neuron (for example, SV2) and an acetylcholine receptor expressed by a myotube cell, for example, by immunostaining or microscope observation.

An artificial neuromuscular junction preferably comprises motor neuron, muscle cells (myotubes), and Schwann cells. An artificial neuromuscular junction may comprise cells other than the above-described cells as long as the function as the artificial neuromuscular junction is maintained.

Motor neuron can be identified using, as an index, the positivity of one or more markers selected from HB9, Islet1, or ChAT.

Muscle cells can be identified by using, as an index, the positivity of one or more markers selected from myosin heavy chain (MHC) or MEF2c.

Schwann cells can be identified using, as an index, the positivity of S-100 marker or the like.

The proportion of motor neurons, muscle cells, and Schwann cells of an artificial neuromuscular junction is not particularly limited as long as the function as the neuromuscular junction can be maintained.

### <Pluripotent Stem Cell>

The term "pluripotent stem cell" herein means a stem cell having pluripotency capable of being differentiated into a variety of cells existing in a living body and also having proliferative capacity, and includes any cells induced to intermediate mesoderm cells used in the present invention. Examples of pluripotent stem cells include, but are not limited to, embryonic stem (ES) cells, embryonic stem (ntES) cells derived from cloned embryos obtained by nuclear transfer, sperm stem cells ("GS cells"), embryonic germ cells ("EG cells"), induced pluripotent stem (iPS) cells, and pluripotent cells derived from cultured fibroblasts and bone marrow stem cells (Muse cells). Preferred pluripotent stem cells are iPS cells, and more preferably human iPS cells, from the viewpoint that they can be obtained without destroying embryos, eggs and the like in the production process.

Methods for producing iPS cells are known in the art, and iPS cells can be produced by introducing an initialization factor into any somatic cell. Here, examples of the initialization factor include a gene or a gene product such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, or Glis1, and these initialization factors may be used singly or in combination. Examples of combinations of the initialization factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26: 2467-2474, Huangfu D, et al. (2008), Nat. Biotechnol. 26: 1269-1275, Shi Y, et al. (2008), Cell Stem Cell, 3, 568-574, Zhao Y, et al. (2008), Cell Stem Cell, 3: 475-479, Marson A, (2008), Cell Stem Cell, 3, 132-135, Feng B, et al. (2009), Nat. Cell Biol. 11: 197-203, R. L. Judson et al., (2009), Nat. Biotechnol., 27: 459-461, Lyssiotis CA, et al. (2009), Proc Natl Acad Sci U S A. 106: 8912-8917, Kim JB, et al. (2009), Nature. 461: 649-643, Ichida JK, et al. (2009), Cell Stem Cell. 5: 491-503, Heng JC, et al. (2010), Cell Stem Cell. 6: 167-74, Han J, et al. (2010), Nature. 463: 1096-100, Mali P, et al. (2010), Stem Cells. 28: 713-720, and Maekawa M, et al. (2011), Nature. 474: 225-9.

Somatic cells include, but are not limited to, any of fetal (fetal) somatic cells, neonatal (neonatal) somatic cells, and mature healthy or diseased somatic cells and any of primary cultured cells, subcultured cells, and cell lines. Specific examples of somatic cells include: (1) tissue stem cells (somatic stem cells) such as neural stem cells, hematopoietic stem cells, mesenchymal stem cells, or dental pulp stem cells; (2) tissue progenitor cells; and (3) differentiated cells such as blood cells (such as peripheral blood cells or cord blood cells), lymphocytes, epithelial cells, endothelial cells, muscle cells, fibroblasts (such as skin cells), hair cells, hepatocytes, gastric mucosal cells, intestinal cells, splenocytes, pancreatic cells (such as exocrine pancreatic cells), brain cells, lung cells, kidney cells, or fat cells.

When iPS cells are used as the cell material for transplantation, it is desirable to use somatic cells having the same or substantially the same HLA genotype of a recipient individual from the viewpoint that rejection does not occur. Here, genotypes are "substantially the same" means that HLA genotypes match to such an extent that an immune response can be suppressed by an immunosuppressant for transplanted cells. For example, somatic cells having an HLA type in which three loci of HLA-A, HLA-B, and HLA-DR or four loci with HLA-C added thereto are identical are "substantially the same".

### Transiently Expressing MyoD in Pluripotent Stem Cells to Induce Myogenic Differentiation

Examples of MyoD to be used in the present invention include human myogenic differentiation 1 (MYOD1) consisting of the amino acid sequence represented by SEQ ID NO: 2 and orthologs thereof in other mammals, and transcriptional and splicing variants thereof. Alternatively, MyoD to be used in the present invention may be a protein having 90% or more, and preferably 95% or more, and more preferably 97% or more amino acid identity with any of the above-described proteins, and having a function (such as activation of transcription of muscle-specific promoter) equivalent to that of the protein.

Examples of nucleic acid encoding MyoD to be used in the present invention include human myogenic differentiation 1 (MYOD1) cDNA consisting of the nucleotide sequence represented by nucleotides 221 to 1183 of SEQ ID NO: 1 and orthologs thereof in other mammals, and transcriptional and splicing variants thereof. Alternatively, MyoD to be used in the present invention may be a nucleic acid encoding a protein having 90% or more, and preferably 95% or more, and more preferably 97% or more nucleotide identity with any of the above-described nucleic acids, and having a function (such as activation of transcription of muscle-specific promoter) equivalent to that of a protein encoded by the nucleic acid. Alternatively, the nucleic acid encoding MyoD may be a nucleic acid having a sense strand that can hybridize with a complementary strand of any of the above-described nucleic acids under stringent conditions. Here, stringent conditions can be determined based on the melting temperature (Tm) of a nucleic acid binding a complex or a probe, as demonstrated in Berger and Kimmel (1987, Guide to Molecular Cloning Techniques Methods in Enzymology, Vol. 152, Academic Press, San Diego CA). Examples of washing conditions after hybridization include "0.1 × SSC, 0.1% SDS, 60°C", and it is preferable that a hybridized state is maintained even after washing under such conditions.

A nucleic acid encoding MyoD may be DNA or RNA, or may be a DNA/RNA chimera. The nucleic acid may be single-stranded, or may be a double-stranded DNA, a double-stranded RNA, or a DNA: RNA hybrid. The nucleic acid is preferably a double-stranded DNA or a single-stranded RNA. The RNA may be RNA into which 5-methylcytidine and pseudouridine (TriLink Biotechnologies) has been incorporated to suppress degradation, or may be RNA modified by phosphatase treatment.

The method for transiently expressing MyoD in pluripotent stem cells is not particularly limited, and for example, the following method can be used. Here, the term "expression", in the case of a nucleic acid encoding MyoD, means production of a MyoD protein by transcription and translation from the nucleic acid in a cell, and in the case of MyoD protein, is synonymous with introduction of the protein into a cell.

When MyoD is in the form of DNA, for example, a vector such as a virus, a plasmid, or an artificial chromosome can be introduced into a pluripotent stem cell by a technique such as lipofection, liposome, or microinjection. Examples of the virus vector include a retrovirus vector, a lentivirus vector, an adenovirus vector, an adeno-associated virus vector, and a Sendai virus vector. Examples of the artificial chromosome vector include a human artificial chromosome (HAC), a yeast artificial chromosome (YAC), and a bacterial artificial chromosome (BAC, PAC). Examples of the plasmid include plasmids for mammalian cells.

A vector can comprise a regulatory sequence such as a promoter, an enhancer, a ribosome binding sequence, a terminator, or a polyadenylation site in such a manner that DNA encoding MyoD can be expressed, and, if necessary, can comprise a selection marker sequence of a drug resistance gene (such as a kanamycin resistance gene, a ampicillin resistance gene, or a puromycin resistance gene), a thymidine kinase gene, a diphtheria toxin gene, or the like, a reporter gene sequence of a fluorescent protein, a β-glucuronidase (GUS), a FLAG, or the like, or the like. Examples of the promoter include an SV40 promoter, an LTR promoter, a CMV (cytomegalovirus) promoter, an RSV (Rous sarcoma virus) promoter, a MoMuLV (Moloney mouse leukemia virus) LTR, an HSV-TK (herpes simplex virus thymidine kinase) promoter, an EF-α promoter, a CAG promoter, and a TRE promoter (CMV minimal promoter with a Tet response element with seven consecutive tetO sequences).

In order to transiently express MyoD, a MyoD gene may be introduced into pluripotent stem cells using a transient expression vector, and in order to more strictly control the expression, it is preferable to use an inducible expression system. Examples of the inducible expression system include a drug induced expression system using tetracycline or a derivative thereof (such as doxycycline), and it is preferable to use a pluripotent stem cell into which a gene construct capable of drug-induced expression of MyoD has been introduced in advance.

For example, when the above-described TRE promoter is used, it is desirable to simultaneously express a fusion protein with tetR and VP16AD or a fusion protein (rtTA) with reverse tetR (rtetR) and VP16AD in an identical cell. Although, in the Tet-On system, when no drug is present, the fusion protein does not bind to TRE, and transcription does not occur, by adding a drug, the fusion protein binds to a TRE promoter and transcription occurs, and therefore, MyoD can be transiently expressed during addition of the drug.

The above-described vector may, in order to incorporate an expression cassette composed of a promoter and DNA encoding MyoD that binds to the promoter into the chromosome of a pluripotent stem cell, and further to excise the cassette as necessary, include a transposon sequence before and after the expression cassette. The transposon sequence is not particularly limited, and examples thereof include piggyBac. In another aspect, the above-described vector may include a LoxP sequence before and after an expression cassette for the purpose of removing the expression cassette.

When MyoD is in the form of RNA, MyoD may be introduced into pluripotent stem cells by a technique such as electroporation, lipofection, or microinjection. When MyoD is in the form of a protein, MyoD may be introduced into pluripotent stem cells by a technique such as lipofection, fusion with a cell membrane permeable peptide (such as TAT and polyarginine derived from HIV), or microinjection.

The period during which MyoD is transiently expressed in pluripotent stem cells to induce muscle cells may be any period as long as myogenic differentiation is sufficiently carried out, and can be appropriately changed depending on the type and properties of pluripotent stem cells to be used, for example, about from 4 to 20 days, preferably about from 4 to 12 days, and more preferably about from 6 to 10 days. For example, when using the above-described drug-induced expression system, it is preferable to add a drug and culture during this period. Examples of other aspects include: when a vector having a transposon sequence is used, a method for stopping expression by introducing transposase into cells after the elapse of the above-described period; and when a vector having a LoxP sequence is used, a method for stopping expression by introducing Cre into cells after the elapse of a desired period.

On the other hand, when MyoD is RNA or a protein, introduction may be carried out a plurality of times in such a manner that MyoD is present in a cell during the above-described period.

The culture condition of a pluripotent stem cell when muscle cells are induced in a state where MyoD is transiently expressed in the pluripotent stem cell is preferably an adhesion culture condition. For example, it is preferable to use a culture dish coated with a cell adhesion molecule of an extracellular matrix or the like, such as Matrigel (BD), type I collagen, type IV collagen, gelatin, laminin, heparan sulfate proteoglycan, or entactin, and a combination thereof and culture in a medium obtained by adding a serum or a serum replacement to a medium used for culturing animal cells as a basal medium. Here, examples of the basal medium include Glasgow Minimum Essential Medium (GMEM), Iscove's Modified Dulbecco's Medium (IMDM), 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixed medium thereof. Examples of serum substitutes include albumin, transferrin, fatty acids, insulin, collagen precursors, trace elements, Knockout Serum Replacement (KSR) (serum replacement of FBS during ES cell culture), ITS-supplements and a mixture thereof.

Preferred conditions for inducing differentiation are those in which pluripotent stem cells adhered to a culture dish coated with Matrigel are cultured in an αMEM medium comprising 10% KSR.

The culture temperature is not particularly limited, and is about 30 to 40°C, and preferably about 37°C. The culture is carried out in an atmosphere of CO₂-containing air, and the CO₂ concentration is preferably about from 2 to 5%.

Induction of myogenic differentiation can be confirmed by the presence of a muscle marker such as MHC or MEF2c. A cell population comprising muscle cells thus produced may be not a single cell population but a cell population comprising other types of cells.

### Culturing Cells Obtained in Step (i) in Medium Comprising Neurotrophic Factor to Induce Neural Differentiation

### <Neurotrophic Factor>

The neurotrophic factor is a ligand for a membrane receptor that plays an important role in survival and function maintenance of motor neurons, and examples thereof include Nerve growth factor (NGF), Brain-derived neurotrophic factor (BDNF), Neurotrophin 3 (NT-3), Neurotrophin 4/5 (NT-4/5), Neurotrophin 6 (NT-6), basic FGF, acidic FGF, FGF-5, Epidermal growth factor (EGF), Hepatocyte growth factor (HGF), Insulin, Insulin-like growth factor 1 (IGF 1), Insulin-like growth factor 2 (IGF 2), Glia cell line-derived neurotrophic factor (GDNF), TGF-b2, TGF-b3, Interleukin 6 (IL-6), Ciliary neurotrophic factor (CNTF), and LIF. Preferred neurotrophic factors in the present invention are NT-3, GDNF, and BDNF. The neurotrophic factor is commercially available, for example, from Wako, R&D systems, or the like, and is readily available, or alternatively, may be obtained by forcibly expressing such a factor in a cell by a method known to those skilled in the art.

The concentration of NT-3 in a culture solution can be, for example, from 0.1 ng/mL to 100 ng/mL, and preferably from 1 ng/mL to 50 ng/mL, and more preferably from 5 ng/mL to 20 ng/mL.

The concentration of GDNF in a culture solution can be, for example, from 0.1 ng/mL to 100 ng/mL, and preferably from 1 ng/mL to 50 ng/mL, and more preferably from 5 ng/mL to 20 ng/mL.

The concentration of BDNF in a culture solution can be, for example, from 0.1 ng/mL to 100 ng/mL, and preferably from 1 ng/mL to 50 ng/mL, and more preferably from 5 ng/mL to 20 ng/mL.

In the present invention, a culture solution to be used in step (ii) can be prepared using a medium used for culturing animal cells as a basal medium. Examples of the basal medium include Glasgow's Minimal Essential Medium (GMEM), IMDM medium, Medium 199, Eagle's Minimum Essential Medium (EMEM), αMEM medium, Dulbecco's modified Eagle's Medium (DMEM), Ham's F12 medium, RPMI 1640 medium, Fischer's medium, Neurobasal Medium (Life Technologies), and a mixed medium thereof. The medium may comprise serum or may be serum-free. If necessary, a medium may comprise, for example, one or more serum replacements such as albumin, transferrin, Knockout Serum Replacement (KSR) (serum replacement for FBS during ES cell culture), N2 supplement (Invitrogen), B27 supplement (Invitrogen), a fatty acid, insulin, a collagen precursor, a trace element, 2-mercaptoethanol, 3'-thiolglycerol, and may also comprise one or more substances such as a lipid, an amino acid, L-glutamine, Glutamax (Invitrogen), a non-essential amino acid, a vitamin, a growth factor, a low molecular weight compound, an antibiotic, an antioxidant, pyruvate, a buffer, or an inorganic salt. A preferred culture medium is a Neurobasal Medium comprising N2 supplement, B27 supplement, NT-3, GDNF, and BDNF.

After inducing myogenic differentiation in step (i), expression of MyoD is stopped, and the medium is changed to a medium for neural differentiation, whereby neural differentiation can be induced.

Regarding culture conditions, the culture temperature is not particularly limited, and is about from 30 to 40°C, and preferably about 37°C, and culturing is carried out in an atmosphere of CO₂-containing air, and the CO₂ concentration is preferably about from 2 to 5%.

The culture period is not particularly limited as long as motor neurons and Schwann cells appear, and it is desirable that step (ii) is carried out for at least 20 days. The culture period is more preferably from 20 days to 120 days, and still more preferably from 30 days to 100 days.

As described above, an artificial neuromuscular junction, i.e., a cell population comprising motor neurons, muscle cells, and Schwann cells can be obtained.

### Kit for Preparing Artificial Neuromuscular Junction from Pluripotent Stem Cells

In another embodiment of the present invention, a kit for producing an artificial neuromuscular junction from pluripotent stem cells is provided. The kit comprises a cell, culture solution, an additive, a culture container, and the like to be used in the above-described steps (i) and (ii). Examples of the kit include a kit comprising one or more reagents selected from the group consisting of pluripotent stem cells with MyoD introduced in an inducible state, a drug such as tetracycline or a derivative thereof, a neurotrophic factor(s), a culture vessel coated with an extracellular matrix, and a basal medium. The kit may further comprise a document or instruction describing a procedure of a manufacturing process.

### Cell Culture Comprising Neuromuscular Junction

The artificial neuromuscular junction of the present invention can be obtained as a cell culture comprising motor neurons, muscle cells, and Schwann cells.

A cell culture comprising such a neuromuscular junction is useful as a model system for a pathological condition (such as myasthenia gravis, Lambert-Eaton syndrome, Miller Fisher syndrome, congenital myasthenia syndrome, or spinal muscular atrophy) caused by disorder (dysfunction or hypoplasia) of a neuromuscular junction. Therefore, such a cell culture product can be used, for example, as a cell preparation for treating a pathological condition caused by a disorder of a neuromuscular junction or a screening system for a therapeutic agent for such a pathological condition.

The present invention provides a pharmaceutical composition (cell preparation) comprising an artificial neuromuscular junction obtained by the method described above, a therapeutic agent for a pathological condition caused by a disorder of a neuromuscular junction, comprising the artificial neuromuscular junction, and a method for treating a pathological condition caused by a disorder of a neuromuscular junction, comprising administering a therapeutically effective amount of the artificial neuromuscular junction, respectively.

Examples of a method of administering a therapeutic agent to a patient in need of treatment include a method of locally administering a resulting artificial neuromuscular junction (cell culture comprising muscle cells and Schwann cells) to an affected part by injection or the like. The number of cells in a cell culture comprised in a therapeutic agent is appropriately adjusted according to the degree of a disease or the like. In use of a pharmaceutical composition (cell preparation), dimethyl sulfoxide (DMSO), serum albumin, or the like may be comprised in the cell preparation to protect the cells, and an antibiotic or the like may be comprised in the cell preparation to prevent bacterial contamination and proliferation. Other pharmaceutically acceptable components (such as a carrier, an excipient, a disintegrant, a buffer, an emulsifier, a suspending agent, a soothing agent, a stabilizer, a preservative, an antiseptic, and physiological saline) may further be comprised in a cell preparation.

### Screening Method or Evaluation Method of Drug

The present invention provides a method for screening or evaluating a therapeutic agent for a disease caused by a disorder of a neuromuscular junction, comprising: adding a test substance to the cell population comprising motor neurons, muscle cells and Schwann cells obtained as described above, and culturing the cell population; and
after culturing, evaluating muscle contraction or calcium concentration in the cells.

Examples of the test substance include a cell extract, a cell culture supernatant, a microbial fermentation product, a marine organism-derived extract, a plant extract, a purified or crude protein, a peptide, a non-peptide compound, a synthetic small molecule compound, and a natural compound.

Such a test substance can also be obtained using any of many approaches in combinatorial library methods known in the art, including (1) a biological library, (2) a synthetic library method using deconvolution, (3) a "one-bead one-compound" library method, and (4) a synthetic library method using affinity chromatography selection. Application of a biological library method using affinity chromatography sorting is limited to peptide libraries, but the other four approaches are applicable to small molecule compound libraries of peptides, non-peptide oligomers, or compounds (Lam (1997) Anticancer Drug Des. 12: 145-67). Examples of methods for synthesizing molecular libraries can be found in the art (DeWitt et al. (1993) Proc. Natl. Acad. Sci. USA 90: 6909-13; Erb et al. (1994) Proc. Natl. Acad. Sci. USA 91: 11422-6; Zuckermann et al. (1994) J. Med. Chem. 37: 2678-85; Cho et al. (1993) Science 261: 1303-5; Carell et al. (1994) Angew. Chem. Int. Ed. Engl. 33: 2059; Carell et al. (1994) Angew. Chem. Int. Ed. Engl. 33: 2061; and Gallop et al. (1994) J. Med. Chem. 37: 1233-51). Compound libraries can be made as solution (see Houghten (1992) Bio/Techniques 13: 412-21) or beads (Lam (1991) Nature 354: 82-4), chips (Fodor (1993) Nature 364: 555-6), bacteria (U.S. Pat. No. 5,223,409), spores (U.S. Patent Nos. 5,571,698, 5,403,484, and 5,223,409), plasmid (Cull et al. (1992) Proc. Natl. Acad. Sci. USA 89: 1865-9), or phage (Scott and Smith (1990) Science 249: 386-90; Devlin (1990) Science 249: 404-6; Cwirla et al. (1990) Proc. Natl. Acad. Sci. USA 87: 6378-82; Felici (1991) J. Mol. Biol. 222: 301-10; and U.S. Patent Application No. 2002103360).

A substance that enhances muscle contraction or calcium concentration compared to a control such as a solvent can be selected or evaluated as a potential therapeutic drug for a disease caused by a disorder of a neuromuscular junction.

Another aspect of the method for screening or evaluating a drug of the present invention is
a method for screening or evaluating a therapeutic agent for a disease caused by a disorder of neuromuscular junction, comprising a process for producing an artificial neuromuscular junction, said process comprising the steps of (i) transiently expressing MyoD in pluripotent stem cells to induce myogenic differentiation, and (ii) inducing neural differentiation by culturing the cells obtained in the step (i) in a medium comprising a neurotrophic factor(s), in which a test substance exists in the step(s) (i) and/or (ii), and in which the test substance is evaluated by analyzing morphology or cell composition of the obtained artificial neuromuscular junction.

For example, an artificial neuromuscular junction is produced using iPS cells from a patient with a disease caused by a disorder of a neuromuscular junction, and when formation of the artificial neuromuscular junction is insufficient without addition of a test substance, and the addition of the test substance improves the formation of the artificial neuromuscular junction, the substance may be a therapeutic for the disease caused by the disorder of the neuromuscular junction of the disease.

### Examples

Hereinafter, the present invention will be described more specifically with reference to Examples, but aspects of the present invention are not limited thereto.

### Material and Method

### Cell Line

MyoD-201B7 was constructed by transforming a 201B7 human iPS cell line with a doxycycline (DOX) inducible MYOD1-expressing piggy bac vector as described in Tanaka et al., PLOS ONE 2013, Volume 8, Issue 4, e61540. MYOD1 was expressed together with mCherry protein and rtTA, and expression of MYOD1 was detected by luminescence of the mCherry protein.

### Differentiation of Neuromuscular Junction

MyoD-201B7 strain was subjected to myogenic differentiation according to the procedure described in Tanaka et al., PLOS ONE 2013. Specifically, MyoD-201B7 cells were seeded on a Matrigel-coated plate, and myogenic differentiation was induced by adding 1 µg/mL of doxycycline to myogenic differentiation medium (10% KSR + αMEM). Ten days after the addition of doxycycline, neural differentiation was induced by changing the medium to Neurobasal medium supplemented with neurotrophic factors (BDNF, GDNF, and NT3; 10 ng/ml each, R&D Systems). The medium was then replaced every 3 to 4 days to continue culturing.

### Flow Sorting of mCherry Positive Cells

On the second day of the myogenic differentiation, mCherry positive cells were sorted out using FACS Aria BD and FACS Diva software. The sorted cells were replated on a Matrigel-coated 96-well plate (8.8 × 10⁴ cells/well), and cultured in myogenic differentiation medium with or without doxycycline. On day 20, the ratio of myotubes to neurons was quantified by immunocytochemistry.

### Motion Vector Analysis

Using α7s ILCE-7S (Sony Corporation), phase-contrast images using a red-filtered optical source and fluorescence (Fluo8, green) were continuously captured at 15 fps. Using the SI8000 Cell Motion Imaging System (Sony Corporation), which provides motion vector analysis based on the block matching method, motion quantification with the red-separated images in regions of interest (ROIs) and brightness analysis with the green-separated images were performed.

Both moving image capture and motion analysis were performed at 38 fps using the SI8000 Cell Motion Imaging System (Sony Corporation).

### Calcium Imaging

MyoD-201B7 cells were seeded at a density of 2.2 × 10⁴ cells/well and differentiated into NMJs in a Costar 96-well black-wall/clear-bottom plate. On day 20, the growth medium was removed, and 100 µL Fluo-8 recording medium was added to the cells at 37°C for 1 hour. The cells were washed twice with PBS and then imaged on In Cell Analyzer 2000. The fluorescence intensity was expressed as the F/F0 ratio.

### Analysis by FDSS (Hamamatsu Photonics K.K.)

MyoD-201B7 cells were seeded onto a Matrigel-coated, 96-well black-wall/clear-bottom plate at a density of 2.2 × 10⁴ cells/well. Half of the medium in each well was exchanged every day until the cultures started contracting. The growth medium was removed, and cells were loaded with 100 µL Fluo-8 recording medium at 37°C for 1 hour. The cells were washed twice with PBS and then the differentiation medium was added to the wells. Ca oscillations were monitored with the FDSS microplate reader, data points were collected at 1-minute intervals, and the data points were analyzed using FDSS software for frequency and amplitude quantification.

### Statistics

Statistical functions of Microsoft Excel 2013 were used for statistical analyses. The results were expressed as mean ± SEM. Statistical significance was determined using Student's t-test and Wilcoxon rank sum test. P < 0.05 was considered significant.

### Transmission Electron Microscopy

NMJ cultures were pre-fixed in 2% paraformaldehyde and 2% glutaraldehyde in 0.1 M phosphate buffer (pH 7.4) at 4°C overnight. Post-fixation was done in 1% osmium tetroxide solution for 1 hour at room temperature. The samples were dehydrated in ethanol (30%, 50%, 70%, 90%, 95%, and 100%) and embedded in Epon resin. Ultrathin sections (80 nm) were cut and stained with uranyl acetate and alkaline lead citrate. The specimens were examined with a transmission electron microscope (H-7650, Hitachi, Ltd.).

### Scanning Electron Microscopy

Samples for SEM observation were pre-fixed in 2% paraformaldehyde and 2% glutaraldehyde at 4°C overnight. Post-fixation was carried out in 1% osmium tetroxide solution for 1 hour at room temperature. The samples were dehydrated in ethanol (30%, 50%, 70%, 90%, 95%, and 100%) assigned a concentration grade and dried in a critical point dryer to remove residues of moisture. The samples were coated with platinum by a cathodic spray method and then imaged with a scanning electron microscope (S-4700, Hitachi, Ltd.).

### Immunocytochemistry (ICC) Labeling

The primary antibodies used were Anti-neurofilament (Millipore number MAB5254, 1:500), anti-synaptic vesicle protein 2 (DSHB number SV2, 1:20), Anti-MHC (Millipore No. A4.1025, 1:1000), anti-Isletl (DSHB No. 40.2D6, 1:100), Anti-HB9 (DSHB No. 81.5C10, 1:100), Anti-ChAT (Millipore No. AB144P, 1:100), and Anti-Tuj1 (Covance No. MMS435P, 1:1000). Complexed anti-BTX-647 (Invitrogen) was used at a concentration of 0.5 ug/ml. Samples were imaged by confocal microscopy (Fluo View-1000, Olympus Corporation, or LSM-710, Carl Zeiss Co., Ltd.).

### Optogenetic Activation

The plasmid deposited at Addgene by Karl Deisseroth was purchased and used as a plasmid containing a photoactivation domain (pLenti-Synapsin-hChR2 (H134R)-EYFP-WPRE, Addgene plasmid number 20945). The lentivirus transduction system (ViraPower hiperform lentivirus expression system, Invitrogen) was used to transduce the target gene into neural cells. NMJ cultures were treated with virus-containing medium at 37°C for 48 hours with 5% CO₂. After virus infection, the medium was removed and the Neurobasal medium was added for target gene expression. The medium was changed every 3 to 4 days. EYFP expression was examined by confocal microscopy with proper optical settings. Photoactivation was performed with at 488-nm laser wavelength. In addition to EYFP signals, bright-field images were captured simultaneously for muscle movement analysis.

### Quantification

Image analysis and quantification were performed by MetaMorph software (Molecular Devices, LLC. USA).

### Results and Discussion

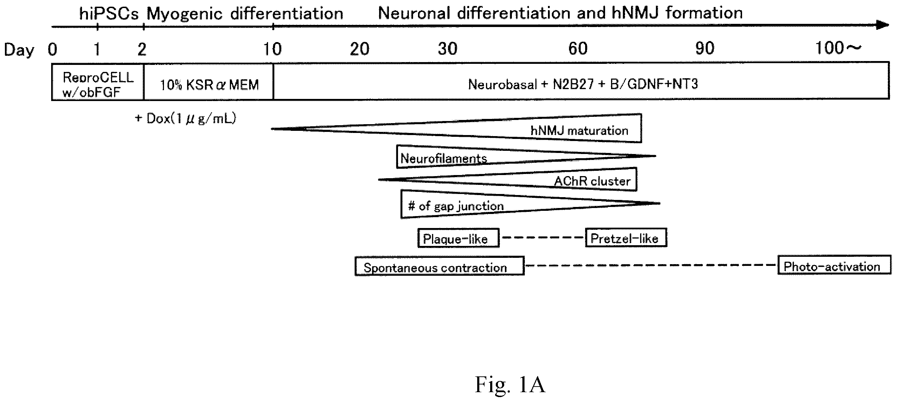

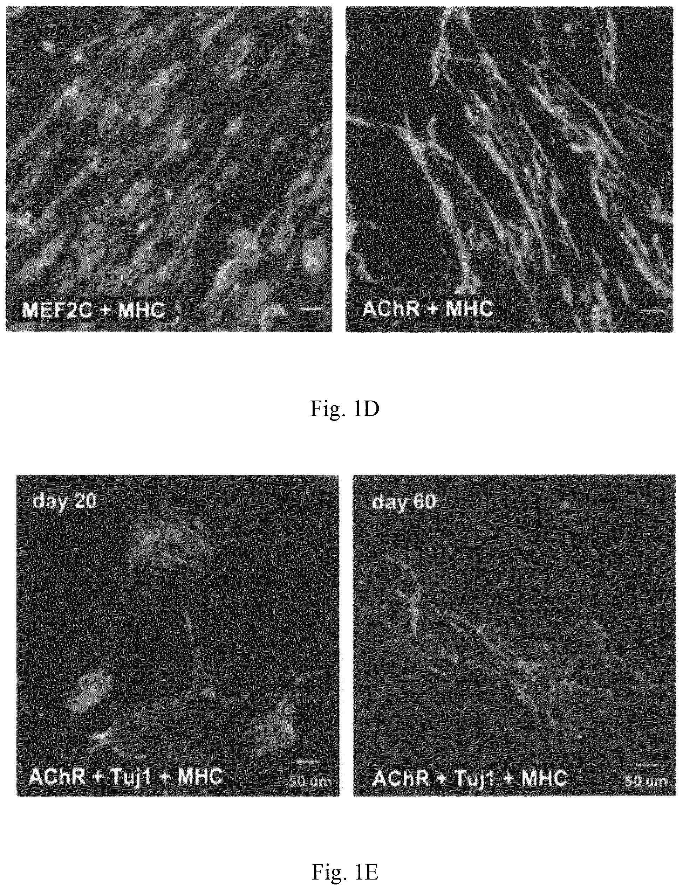

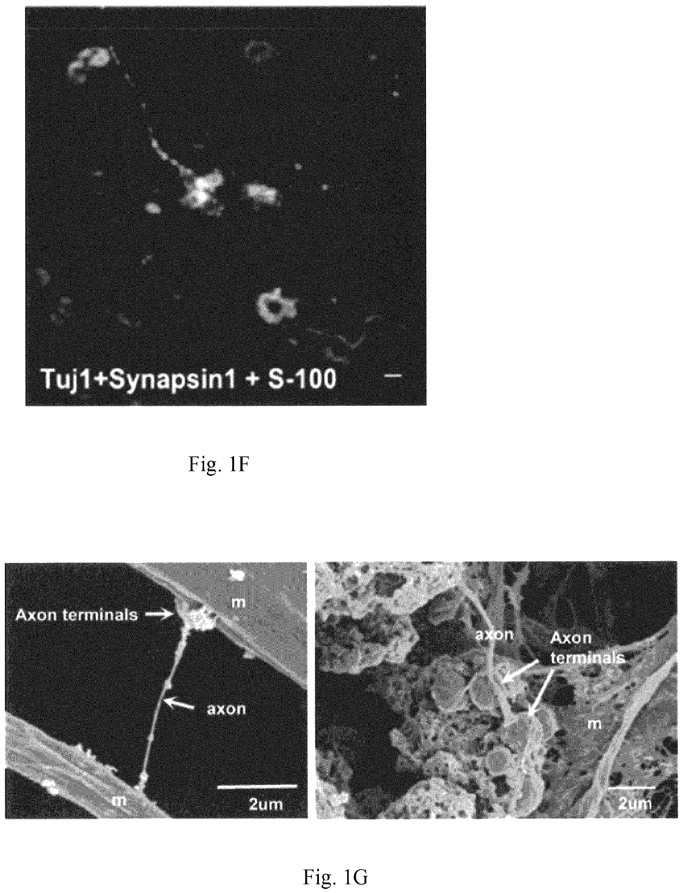

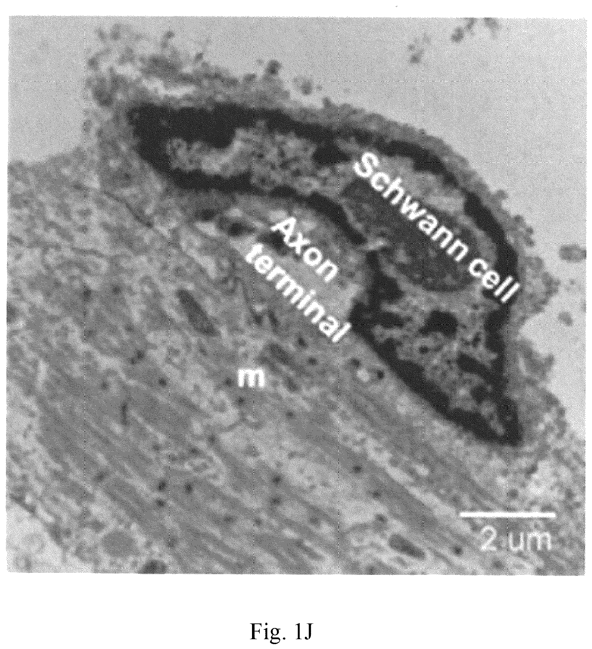

HiPSCs are efficiently induced into mature myotubes by transient expression of MYOD1 by a doxycycline (DOX) inducible vector. By using this method, we first differentiated a 201B7-iPSC clone (201B7^{MYOD}) transfected with a Dox-inducible construct into myotubes. By switching the medium to one suitable for nerve cell differentiation on day 10 (Fig. 1A), AChRs clustering on the surface of myotubes and juxtaposed to the axon terminal were detected on day 20 and NMJ-like structures could be obtained (Fig. 1E). Schwann cells were also detected in the structure (Fig. 1F).

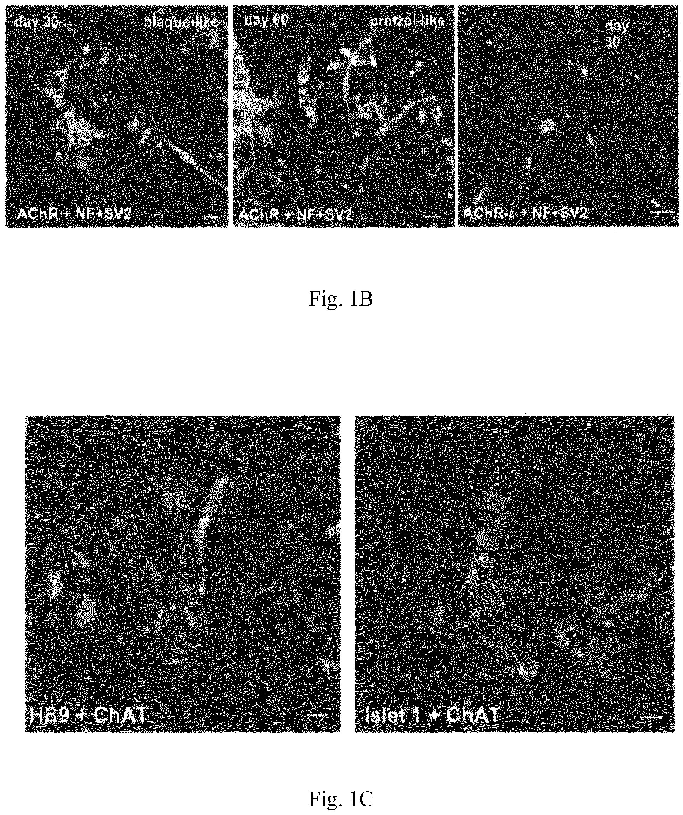

Markers associated with pre- and postsynaptic cell types were detected (Figs. 1C and 1D).

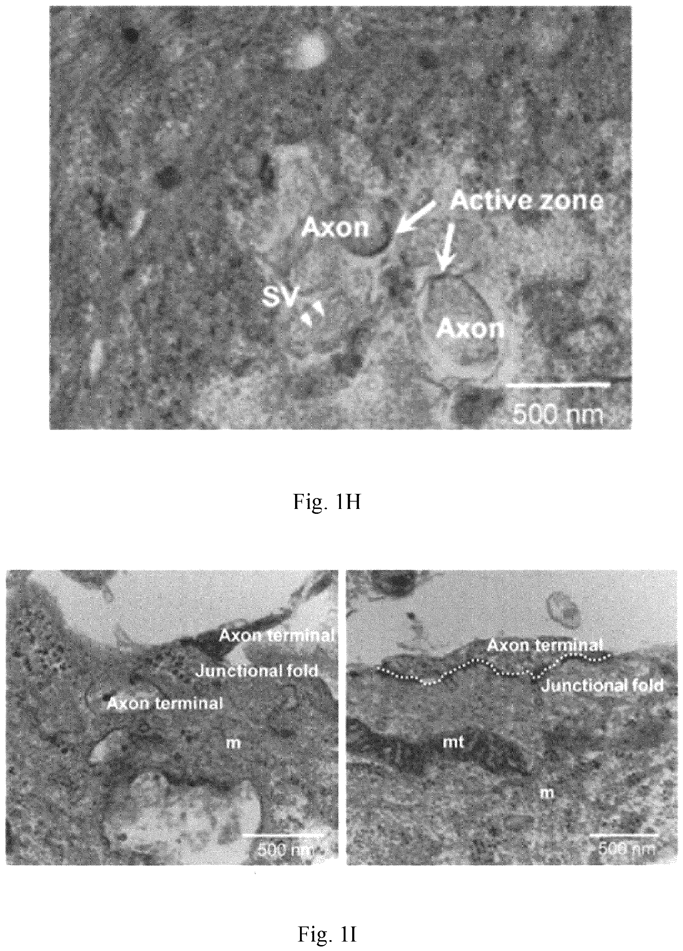

Electron microscopy (EM) images showed swelling of axon terminals binding to the myotubes and Schwann cells capping terminal boutons in NMJ culture (Figs. 1G and 1I).

Synaptic vesicles and mitochondria accumulated at the pre- and postsynaptic sites of NMJs, clearly separated by high electron density active zones (Figs. 1H and I).

From these observations, the *in vitro* NMJ comprised major cell types of NMJ and had the structural characteristics of NMJ.

We then observed whether our hNMJ culture recapitulated the structural and morphological changes of *in vivo* synapse formation. On day 20, a region, occupied by a nerve ending which is a so-called "plaque-like" clustering, whose contours, if not all of them, are clear was formed by AChR, indicating an immature embryonic NMJ. By day 60, the plaque perforated and perfectly aligned with MN axon terminals, giving the junction a "pretzel-like" appearance (Fig. 1B). In parallel with the AChR morphological change, the AChR subunit shifted from γ to ε (Fig. 1B). In the development of NMJs *in vivo,* multiple innervations occur at first, with all but one axonal input to each myotube being removed as a result of neuronal plasticity. In our culture condition, the number of neurons and NMJs decreased in the late stage (Fig. 1E), showing that neural sorting does occur during hNMJ maturation *in vitro.* Through EM image analysis, muscle structural fibers, Z lines, multiply branched axon terminals, and necrotic-like synapse removal processes were observed (Figs. 2a to c). The axon terminals in the matured NMJs had synaptic vesicles clustered inside and showed cytomatrix depositions and active zones juxtaposed to the postsynaptic density (Figs. 2d and e). The protruding space of subneural clefts was surrounded by sarcolemma, where AChRs were localized (Fig. 2f).

As described above, we were able to observe a series of hNMJ developmental stages in the NMJ culture system.

The contribution of a neuronal factor agrin, which accumulates and stabilizes AChRs at synaptic sites, to the *in vitro* NMJ system was investigated. Agrin administration increased the area of the NMJ in long-term culture, while in the presence of antiagrin antibody, only remnants of myotubes and deteriorated axon terminals were left in the culture (Figs. 2g and h). Accordingly, agrin was found to play an important role in the formation and maintenance of hNMJs in our *in vitro* system as well as *in vivo.*

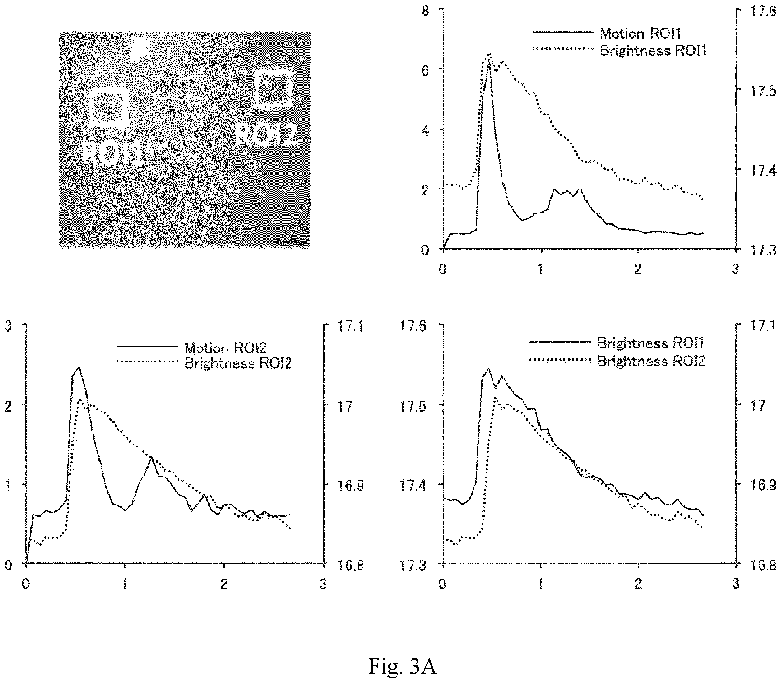

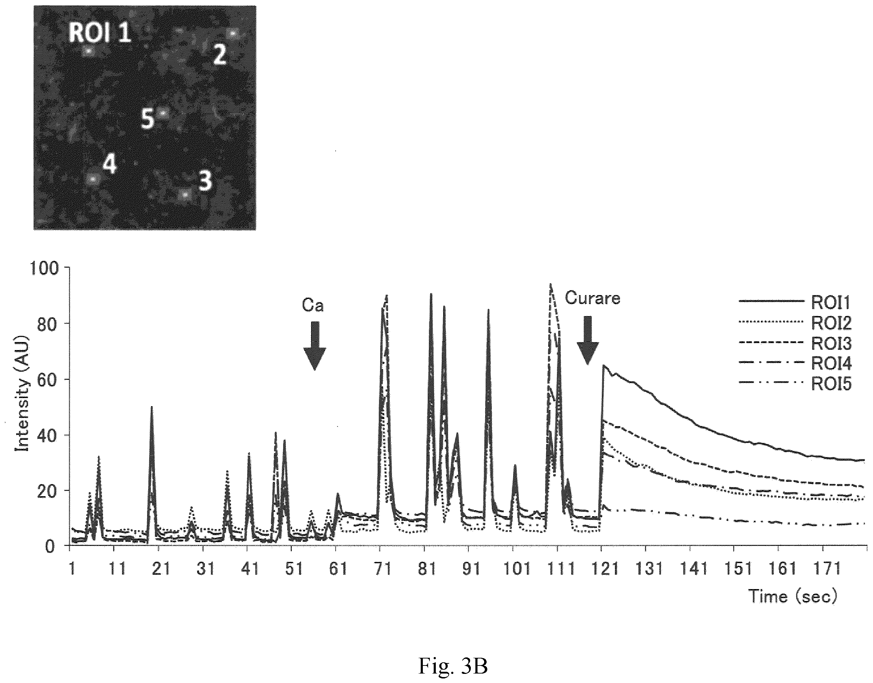

From day 15 to day 25, the entire areas of each culture favorably showed spontaneous, synchronized contraction-like movement. The movement completely synchronized with the intracellular Ca²⁺ concentration of the myotubes, and was inhibited by skeletal muscle relaxant dantrolene, confirming that the movement was actually myotube contractions (Fig. 3A). Further, the excitation of myotubes was enhanced by Ca²⁺, which promotes the release of Ach from neurons, and reduced by curare, a competitive inhibitor of AChR. This indicates that muscle contraction was induced via AChR (Fig. 3B). Therefore, myotube contraction was considered to be governed by spontaneous neuronal activity via functional synaptic connections between the neurons and the myotubes.

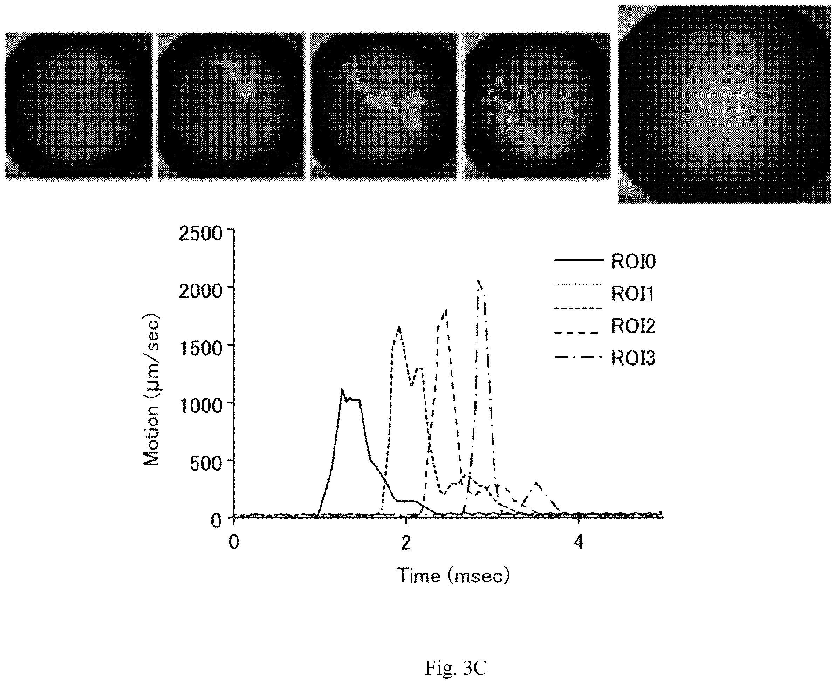

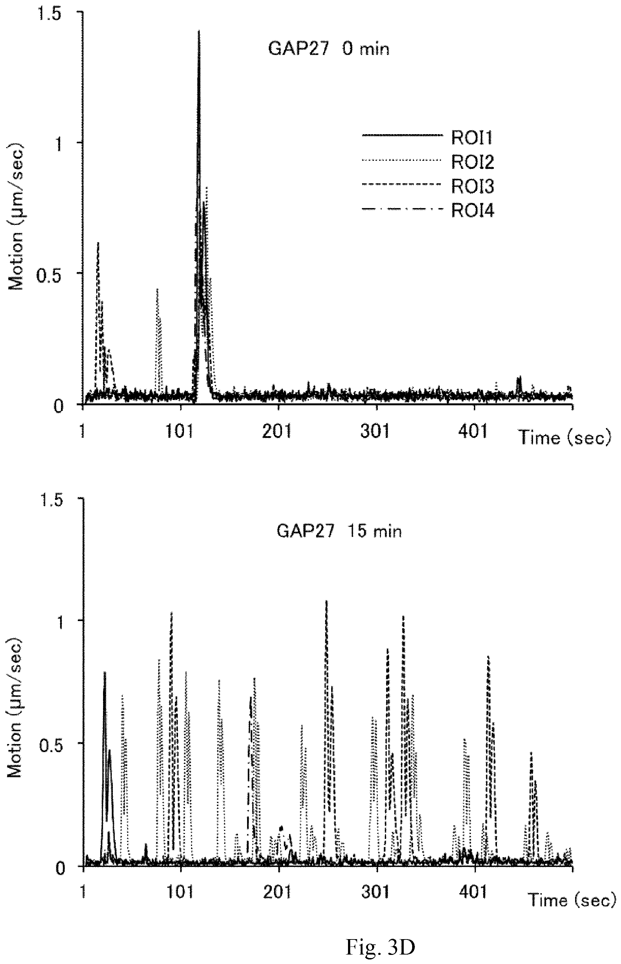

During the development of NMJs, gap junctions, which are intercellular channels between myotubes that enable the cell-cell propagation of electrical activity, are transiently expressed. When we closely observed the contraction by increasing the frame rate of the motion vector analysis, the contractions of the myotubes were not completely synchronized, but propagated to adjacent areas (Fig. 3C). The propagation of the contraction was interrupted by a gap junction inhibitor, gap27, indicating that this contraction was caused by propagation of action potentials through gap junctions (Fig. 3D).

As described above, these functional analyses showed that our *in vitro* NMJs recapitulated key features in the early stages of *in vivo* NMJ development.

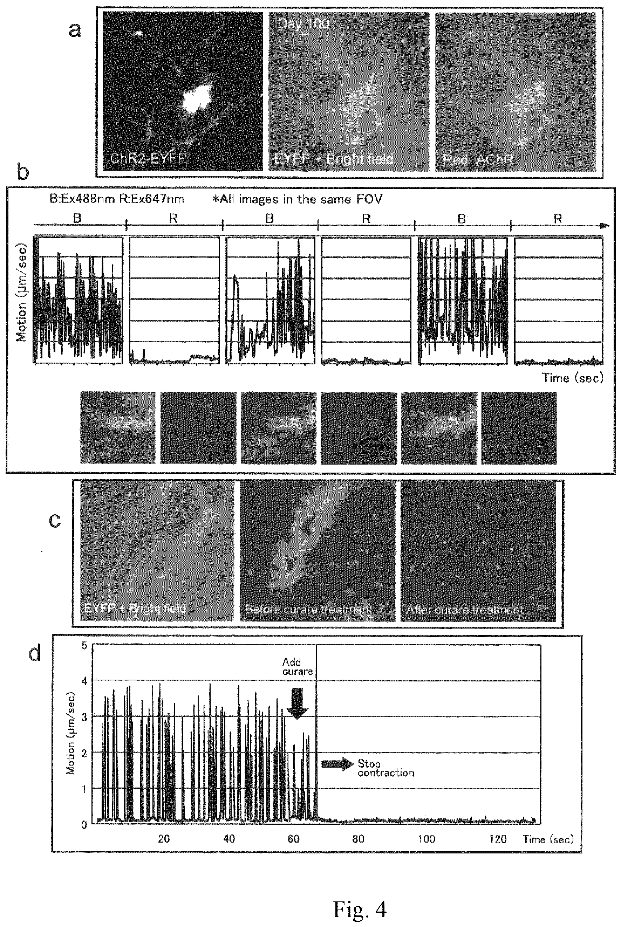

Next, we activated neurons by introducing a photoactivating channel into long-term NMJ culture by lentivirus infection. At day 100 of the NMJ differentiation, molecularly modified neuronal cells of NMJ were visualized by the expression of channelrhodopsin-EYFP (ChR2-EYFP) driven by the synapsin1 promoter (Fig. 4a). Muscle contraction was triggered only by blue light, indicating that the photoactivated system was successfully established (Fig. 4b). No contraction was observed in the wells without ChR2-EYFP even when stimulated with a blue light laser (data not shown). Curare administration to the cultures inhibited contractions, indicating that muscle contractions were actually caused by the Ach-Ach axis in this *in vitro* system (Fig. 4c).

As described above, the optogenetic NMJ system demonstrated the functional maturation of our NMJs in long-term culture.

Next, we confirmed the abundance of motor neuron in NMJ cultures by immunostaining and flow cytometry. Details are as follows.

### Immunostaining and Image Analysis

Medium was removed from NMJ cultures. Washing with PBS for 1 minute was carried out three times, and the cells were fixed with 4% paraformaldehyde/PBS for 10 minutes at room temperature. The NMJ cultures were washed three times with 0.1% BSA/PBS and permeabilized with 4% paraformaldehyde + 0.1% Triton at room temperature for 10 minutes. Rehydration was carried out three times for 5 minutes with 0.1% BSA/PBS, a blocking treatment was carried out for 1 hour at room temperature using 0.1% BSA/PBS + 0.5% Tween-20, cover slips were transferred to Petri dishes provided with a parafilm layer, and drying was carried out. Using a primary antibody (antibody against Islet1 or NeuN) diluted with 50 µl of PBS, a reaction was carried out at room temperature for 1 hour, washing with PBS for 5 minutes was carried out three times, and a reaction using a secondary antibody diluted in PBS was carried out at room temperature for 1 hour (under light shielding), and washing with PBS for 5 minutes was carried out three times. The slides were washed several times with ddH₂O and dried. Five µl of glycerol:PBS (1:1) was added and the slides were observed with a confocal microscope (LSM-710, Carl Zeiss Co., Ltd.). MetaMorph software (Molecular devices, LLC. USA) was used for image analysis.

### Flow Cytometry

Medium was removed from NMJ cultures, the cells were detached with trypsin. washed three times for one minute with PBS, and fixed with 4% paraformaldehyde/PBS for 10 minutes at room temperature. After washing three times with 0.1% BSA/PBS, the cells were permeabilized with 4% paraformaldehyde + 0.1% Triton at room temperature for 5 minutes. Washing with 0.1% BSA/PBS for 1 minute was carried out three times, and a blocking treatment was carried out for 20 minutes at room temperature using 0.1% BSA/PBS. Using a primary antibody diluted with PBS (antibody against Islet1 or HB9), a reaction was carried out at room temperature for 1 hour, washing with PBS was carried out three times, and a reaction using a secondary antibody diluted in PBS was carried out at room temperature for 45 minutes (under light shielding), and washing with PBS for 1 minute was carried out three times. Analysis was carried out using a flow cytometer (Aria 2, BD) and FlowJo software (BD).

### Results

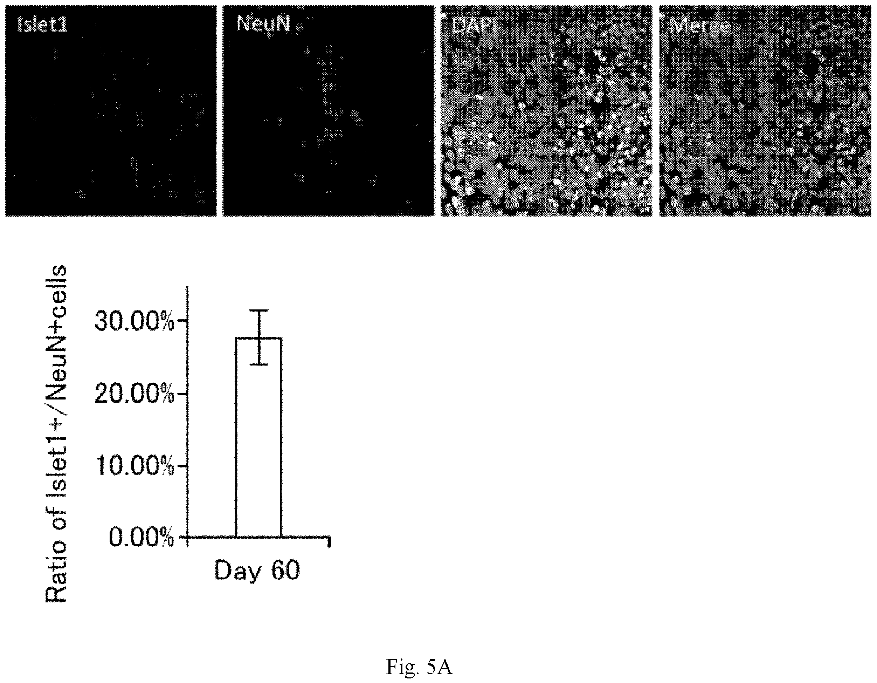

From results of the immunostaining, it was found that motor neurons were recognized from about day 30, and the ratio was 27.4% of the total nerve cells (the ratio of Islet1-positive cells to NeuN-positive cells) on day 60 (Fig. 5A).

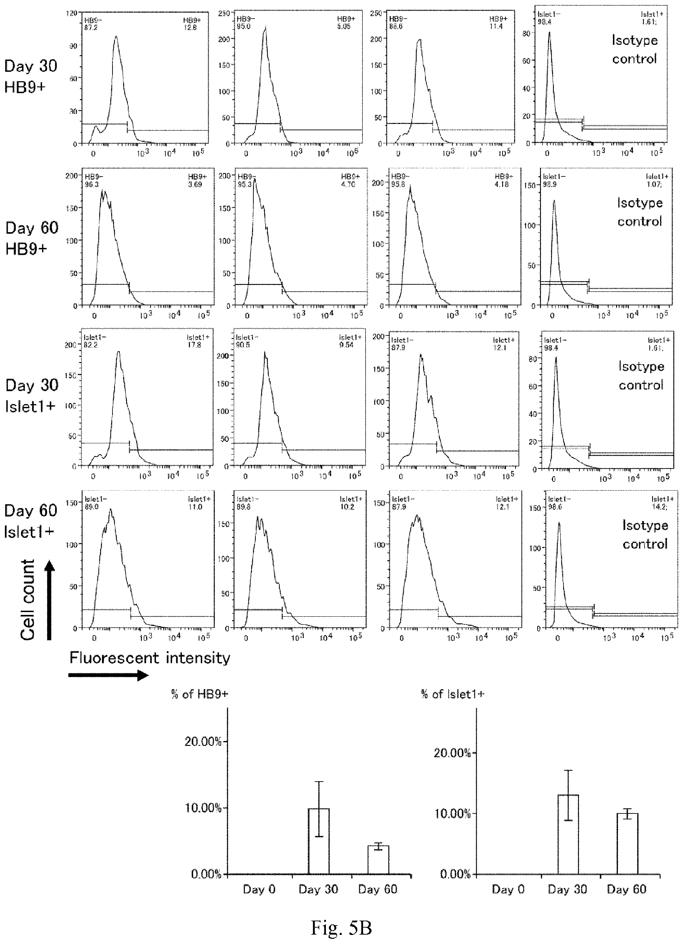

From results of flow cytometry, 13.16% of the total cells were HB9-positive cells and 19.61% were Islet1-positive cells in day 30 culture, and 4.56% of the total cells were HB9-positive cells and 10.84% were Islet1-positive cells in day 60 culture (Fig. 5B).

Next, we prepared cells (201B7^{MYOD}-SMN^{KD} cells) in which SMN (survival of motor neuron) protein of MyoD-201B7 cells was knocked down to construct a pathophysiological model for patients with spinal muscular atrophy, and NMJs were formed by the same method as described above. Details are as follows.

### Western Blot

The SMN protein expression in the prepared 201B7^{MYOD}-SMN^{KD} cells was analyzed by Western blotting.

### Immunostaining

The 201B7^{MYOD}-SMN^{KD} cells on day 30 of culture were immunostained using an antibody to NF + SV2 or AChR as a primary antibody according to the same protocol as the immunostaining described above.

### Scanning Electron Microscopy

Tissues were fixed in 2% paraformaldehyde + 2% glutaraldehyde diluted in PBS (pH 7.4) for 1 hour, washed thoroughly three times in 0.1 M cacodylate buffer for 5 minutes, fixed in 60 minutes in 1% osmium tetroxide diluted in 0.1 M cacodylate buffer, and washed three times in 0.1 M cacodylate buffer for 15 minutes. Dehydration was carried out sequentially with the following grades of ethanol diluted in phosphate buffer: with 30% ethanol for 5 minutes, with 50% ethanol for 5 minutes, with 70% ethanol for 5 minutes, with 90% ethanol for 10 minutes, with 95% ethanol for 15 minutes twice, and with 100% ethanol for 30 minutes three times. The tissues were then dried at the critical point, fixed to a stud, sputter coated, stored in a desiccator, and observed with a scanning electron microscope (S-4700, Hitachi, Ltd.).

### Transmission Electron Microscopy

Sample were pre-fixed in 2% paraformaldehyde + 2% glutaraldehyde diluted in 100 mM phosphate buffer (pH 7.0) for 2-24 hours, post-fixed in 1% osmium tetroxide diluted in 0.1 M cacodylate buffer for 1-2 hours at room temperature, and dehydrated in the following order: with 30% acetone for 15 minutes, with 50% acetone for 15 minutes, with 70% acetone for 15 minutes, with 90% acetone for 15 minutes, and with 100% acetone for 30 minutes three times. Thereafter, resin embedding (twice with acetone for 15 minutes using Epon mix) and resin infiltration (1 hour with 2:1 mixture of acetone: resin, then 1 hour with 1:1 mixture of acetone: resin, 1 hour with 1:2 mixture of acetone: resin, overnight with 100% resin) were carried out, and then the resin was replaced with fresh resin and a treatment was carried out for 1 hour. Then, the sample was embedded in fresh resin in a mold and polymerization was carried out at from 60 to 70°C for 72 hours. The sample was ultrathin sectioned, observed with a transmission electron microscope (H-7650, Hitachi, Ltd.), and the movement analysis of contraction of mature myotubes was carried out. Recording and analysis were carried out using the SI8000 Cell Motion Imaging System (Sony Corporation).

### Results

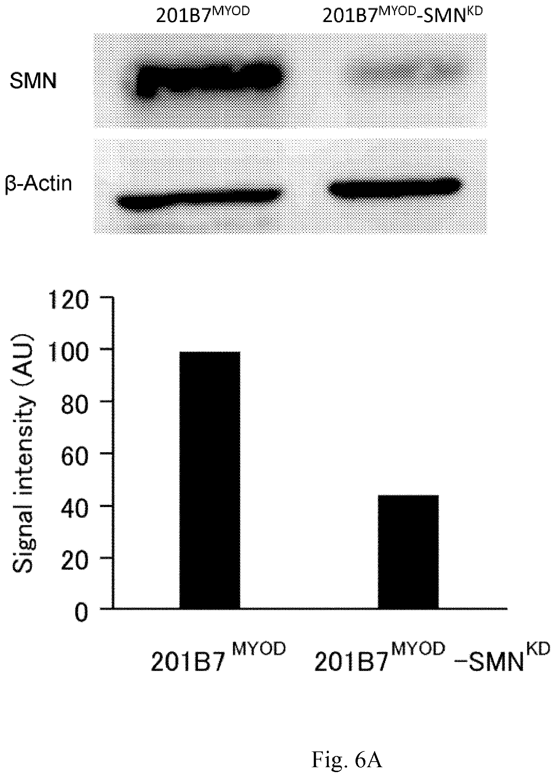

It was found that the SMN protein expression level in 201B7^{MYOD}-SMN^{KD} cells was decreased by about 60% from that of SMN protein non-knockdown 201B7^{MYOD} cells (Fig. 6A).

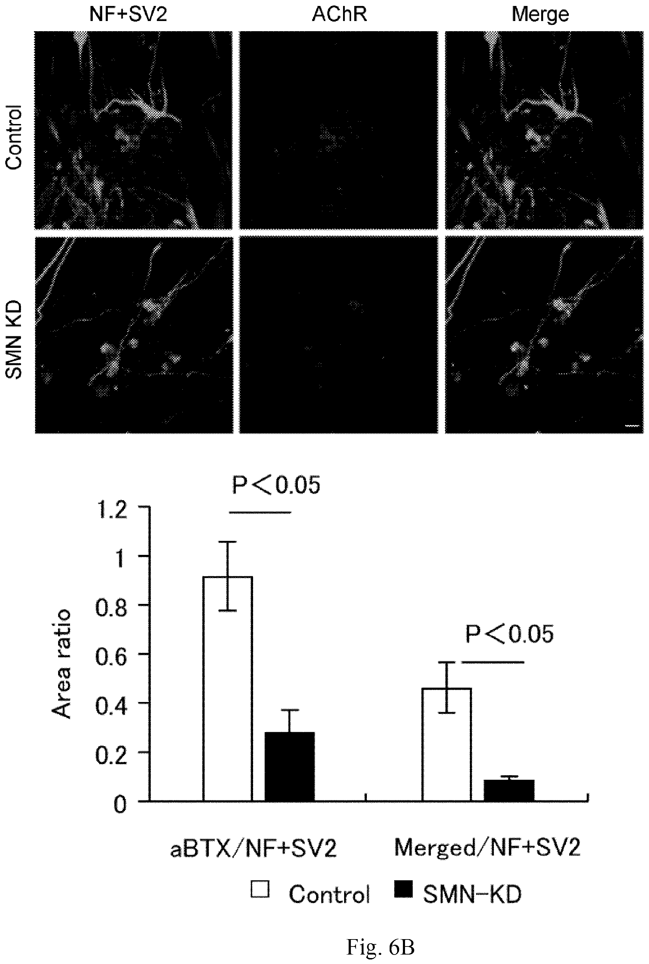

The area of the NMJ in 201B7^{MYOD}-SMN^{KD} cells (SMN KD) was significantly smaller than in 201B7^{MYOD} cells (Control) (Fig. 6B).

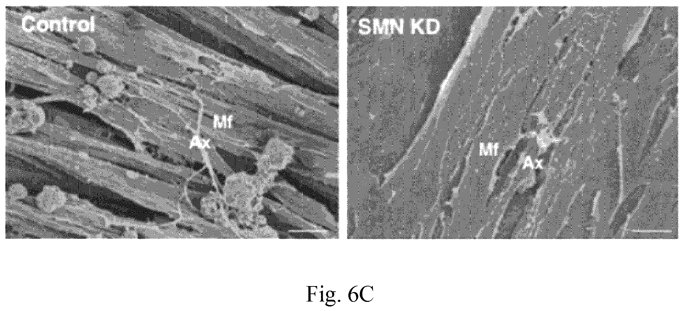

In the SEM images of the 201B7^{MYOD} cell (Control) culture, bunches of myotubes and axons that were well extended were observed, and myotubes anchored to enlarged axon terminals were observed (Fig. 6C).

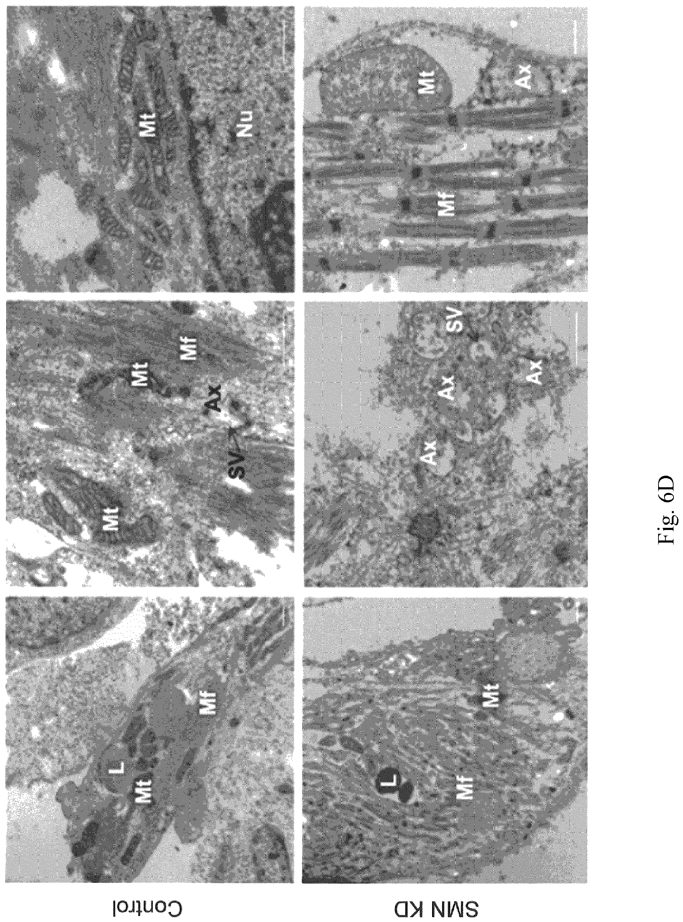

In 201B7^{MYOD}-SMN^{KD} cell (SMN KD) culture, myotubes were flatter and thinner and had fewer axon terminals than in 201B7^{MYOD} cell (Control) culture. The details of intracellular myotube and NMJ-related structures were completely different from the 201B7^{MYOD} cell (Control) culture. The mitochondrial morphology and state in 201B7^{MYOD}-SMN^{KD} cells were damaged, and muscle fibers had less integrity in their structure (Fig. 6D).

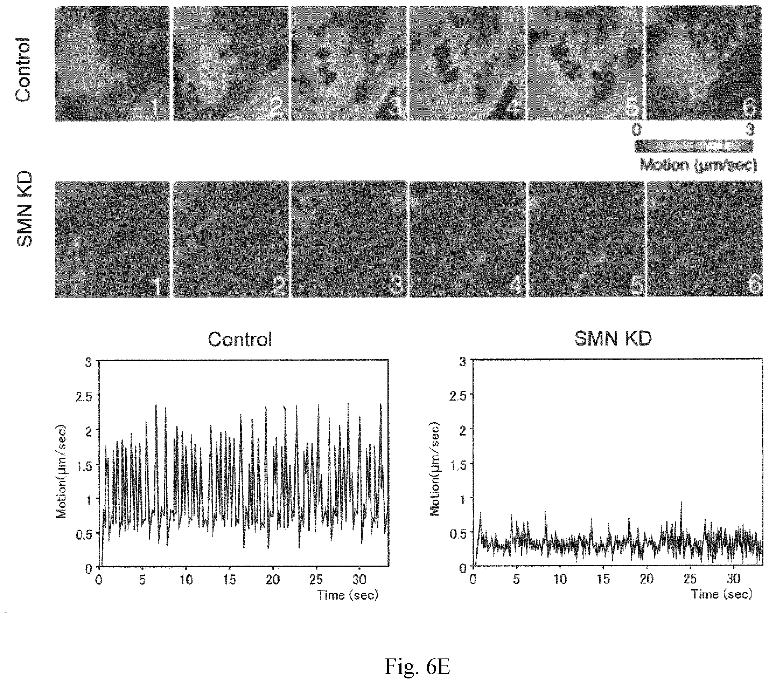

Regarding the amplitude and patterns of NMJ-dependent muscle contractions observed to evaluate functional differences, the area of contractions was smaller in 201B7^{MYOD}-SMN^{KD} cells (SMN KD) than in 201B7^{MYOD} cells (Control). The synchronicity of contraction was disrupted in 201B7^{MYOD}-SMN^{KD} cells (SMN KD), and the maximum speed of muscle contraction was smaller than in 201B7^{MYOD} cells (Control) (Fig. 6E).

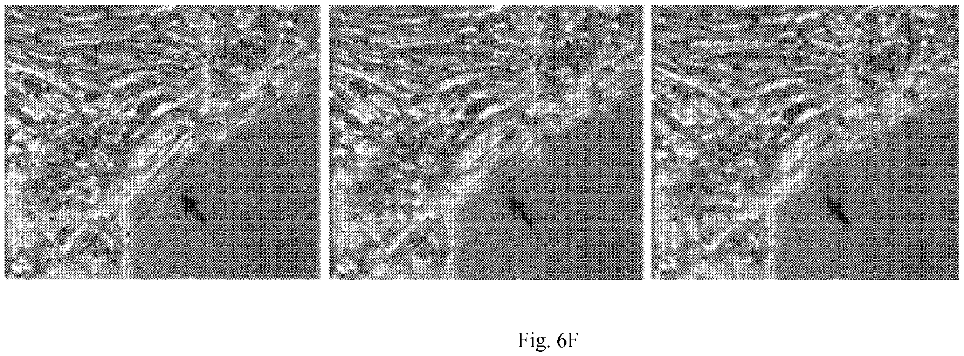

In 201B7^{MYOD}-SMN^{KD} cells, myotubes were often observed to break during muscle contraction, but no fragility was observed in 201B7^{MYOD} cells (Fig. 6F).

This phenomenon is thought to indicate that SMA-myotubes may be susceptible to muscle contraction and is associated with the muscle atrophy seen in SMA patients.

As described above, decrease in the frequency of NMJ formation and weakening of muscle cells were observed, and it was found that the area of muscle cells contracting by stimulation from NMJ was decreased.

Accordingly, it was found that the *in vitro* NMJ induction method of the present invention can be used for disease models and drug evaluation.

According to the *in vitro* NMJ culture method of the present invention, an NMJ composed of three types of cells: skeletal muscle, MN, and Schwann cells differentiated according to a developmental process *in vivo* by a simple two-dimensional culture operation for temporarily expressing MyoD in iPSCs and then inducing neural differentiation. The method of the present invention has an appropriate balance between myogenesis and neurogenesis in NMJ construction, and reproduces complex mutual signals between cells such as nerve, muscle and Schwann cells in NMJ formation and maintenance. Since the obtained NMJ shows the characteristics of a mature NMJ both in appearance and function, it can be used as a platform for a disease model, and contributes to cell therapy, drug effects, or the like for diseases based on a disorder of development or function of NMJ.

## Claims

1. A method for producing an artificial neuromuscular junction, comprising the steps of:
(i) transiently expressing MyoD in pluripotent stem cells to induce myogenic differentiation, and
(ii) culturing the cells obtained in the step (i) in a medium comprising a neurotrophic factor(s) to induce neural differentiation.

2. The method according to claim 1, wherein the transient expression of MyoD in the step (i) is a drug-induced expression.

3. The method according to claim 1 or 2, wherein said neurotrophic factors in the step (ii) are Glial cell line-derived neurotrophic factor (GDNF), Brain-derived neurotrophic factor (BDNF), and Neurotrophin 3 (NT-3).

4. The method according to any one of claims 1 to 3, wherein the step (i) is carried out for from 4 to 20 days.

5. The method according to any one of claims 1 to 4, wherein the step (ii) is carried out for from 20 to 120 days.

6. The method according to any one of claims 1 to 5, wherein the artificial neuromuscular junction comprises motor neurons, muscle cells, and Schwann cells.

7. The method according to any one of claims 1 to 6, wherein the pluripotent stem cells are induced pluripotent stem cells.

8. The method according to claim 7, wherein the pluripotent stem cells are human induced pluripotent stem cells.

9. A cell population comprising: a motor neuron, a muscle cell, and a Schwann cell, each induced from a pluripotent stem cell.

10. A pharmaceutical composition comprising the cell population according to claim 9.

11. A method for screening or evaluating a therapeutic agent for a disease caused by a disorder of a neuromuscular junction, comprising the steps of:
adding a test substance to the cell population according to claim 9 and culturing the cell population, and
after culturing, evaluating muscle contraction or calcium concentration in the cells.

12. A method for screening or evaluating a therapeutic agent for a disease caused by a disorder of a neuromuscular junction, comprising a process for producing an artificial neuromuscular junction, said process comprising the steps of (i) transiently expressing MyoD in pluripotent stem cells to induce myogenic differentiation, and (ii) inducing neural differentiation by culturing the cells obtained in the step (i) in a medium comprising a neurotrophic factor(s),
wherein a test substance exists in the step(s) (i) and/or (ii), and
wherein the test substance is evaluated by analyzing morphology or cell composition of the obtained artificial neuromuscular junction.
